# EUROPEAN PATENT SPECIFICATION

(11) **EP 2 833 906 B1**
(45) Date of publication and mention of the grant of the patent: **12.04.2023**
(21) Application number: 13712728.8
(22) Date of filing: 15.03.2013
(51) Int. Cl.: A61K 38/48, A61P 17/02, A61K 35/612, A61K 38/54

(54) **MIXTURE OF ENZYMES FROM ANTARCTIC KRILL FOR USE IN THE REMOVAL OF A BIOFILM**
ENZYMMISCHUNG VON ANTARKTISCHEM KRILL FÜR DIE ENTFERNUNG VON BIOFILM
MELANGE ENZYMATIQUE DU KRILL ATLANTIQUE POUR ENLEVER DES BIOFILMS

(30) Priority: 05.04.2012 US 201261620893 P
(43) Date of publication of application: 11.02.2015
(73) Proprietor: Marizyme, Inc., Jupiter, FL 33458 (US)
(72) Inventor: RUTMAN, Max, Nuñoa, Santiago (CL); VANSCHEIDT, Wolfgang, 79100 Freiburg (DE); VINCENT, Jan, S-114 42 Stockholm (SE); KOCHINKE, Frank, San Jose, California 95148 (US)
(74) Representative: Dentons Patent Solutions Rechtsanwaltsgesellschaft mbH
(86) International application number: PCT/EP2013/055445
(87) International publication number: WO 2013/149809

(56) References cited:
- EP-A2- 0 107 634
- WO-A1-96/24371
- WO-A1-2008/070698
- WO-A2-2009/060013
- DIRK HOEFER: "A Novel In Situ Self-Dissolving Needle Web Based on Medicated Cellulose Hollow Fibres with Drug Delivery Features", THE OPEN MEDICAL DEVICES JOURNAL, vol. 3, no. 1, 29 December 2011 (2011-12-29), pages 1-8, XP055060738, ISSN: 1875-1814, DOI: 10.2174/1875181401103010001
- HELLGREN KRISTIAN: "Assessment of Krillase chewing gum for the reduction of gingivitis and dental plaque", JOURNAL OF CLINICAL DENTISTRY, PROFESSIONAL AUDIENCE COMMUNICATIONS, YARDLEY, PA, US, vol. 20, no. 3, 2009, pages 99-102, XP009137379, ISSN: 0895-8831 cited in the application
- Stellan Lind: "Krillase shows advantages compared with competing products", , 1 December 1998 (1998-12-01), pages 1-1, XP055060777, Retrieved from the Internet: URL:http://news.cision.com/biophausia/r/kr illase-shows-advantages-compared-with-comp eting-products,c5113 [retrieved on 2013-04-23]
- Stellan Lind: "Patient recruitment for Krillase study completed", , 4 August 1998 (1998-08-04), pages 1-1, XP055060778, Retrieved from the Internet: URL:http://news.cision.com/biophausia/r/pa tient-recruitment-for-krillase--study-comp leted,c3344 [retrieved on 2013-04-23]
- HELLGREN L ET AL: "PROTEASES OF ANTARCTIC KRILL EUPHAUSIA-SUPERBA A NEW SYSTEM FOR EFFECTIVE ENZYMATIC DEBRIDEMENT OF NECROTIC ULCERATIONS", EXPERIENTIA (BASEL), vol. 42, no. 4, 1986, pages 403-404, XP009169081, ISSN: 0014-4754
- I. CECILIA HAHN BERG ET AL: "Proteolytic degradation of oral biofilms in vitro and in vivo: potential of proteases originating from Euphausia superba for plaque control", EUROPEAN JOURNAL OF ORAL SCIENCES, vol. 109, no. 5, 1 October 2001 (2001-10-01), pages 316-324, XP055060893, ISSN: 0909-8836, DOI: 10.1034/j.1600-0722.2001.00099.x cited in the application
- WESTERHOF W ET AL: "PROSPECTIVE RANDOMIZED STUDY COMPARING THE DEBRIDING EFFECT OF KRILL ENZYMES AND A NON-ENZYMATIC TREATMENT IN VENOUS LEG ULCERS", DERMATOLOGICA (BASEL), vol. 181, no. 4, 1990, pages 293-297, XP009169118, ISSN: 0011-9075
- JAMES GARTH A ET AL: "Biofilms in chronic wounds", WOUND REPAIR AND REGENERATION, WILEY-BLACKWELL PUBLISHING, INC, US, vol. 16, no. 1, 2008, pages 37-44, XP002494544, DOI: 10.1111/J.1524-475X.2007.00321.X cited in the application
- DATABASE BIOSIS [Online] BIOSCIENCES INFORMATION SERVICE, PHILADELPHIA, PA, US; 1986, HELLGREN L ET AL: "PROTEASES OF ANTARCTIC KRILL EUPHAUSIA-SUPERBA A NEW SYSTEM FOR EFFECTIVE ENZYMATIC DEBRIDEMENT OF NECROTIC ULCERATIONS", Database accession no. PREV198682037633 & HELLGREN L ET AL: "PROTEASES OF ANTARCTIC KRILL EUPHAUSIA-SUPERBA A NEW SYSTEM FOR EFFECTIVE ENZYMATIC DEBRIDEMENT OF NECROTIC ULCERATIONS", EXPERIENTIA (BASEL), vol. 42, no. 4, 1986, pages 403-404, ISSN: 0014-4754

## Description

The invention concerns a mixture of enzymes from krill for use in the treatment of a disease related to or an inflammatory condition caused by a biofilm formed on a soft tissue of a mammal.

From Hahn Berg, I.C. et al., Eur. J. Oral. Sci. 2001, 109, pages 316 - 324, the proteolytic degradation of oral biofilms in the form of dental plaque, *in vitro* and *in vivo,* using proteases from Antarctic krill, referred to as "Krillase", is known. Plaque is a very specific biofilm forming on the solid surface of teeth which biofilm comprises salivary proteins, a complex microbial community, and a matrix of polymers of bacterial and salivary origin.

From Hellgren, K., J. Clin. Dent. 2009, 20, pages 99 - 102, the use of a Krillase chewing gum for the reduction of gingivitis and dental plaque is known. According to this paper disruption of dental biofilm, together with limiting the bacterial attachment or adherence to oral surfaces, may represent a way to prevent plaque formation and consequently, most periodontal diseases.

US 2005/0025722 A1 discloses the use of a multifunctional enzyme that may be a krill-derived multifunctional enzyme. The enzyme may be used for the treatment of microbial infection such as an infection causing gastrointestinal disease, for the treatment of wound infection or for the treatment of acute or chronic inflammation.

Furthermore, it is known to use a krill enzyme mixture for debridement of wounds to accelerate healing of the wounds. According to WO 2008/063229 A2 which concerns compositions to destroy internal cancerous lesions selectively by the administration of a combination of a debridement protease enzyme and a denaturant of cell structural proteins and/or cell adhesion proteins, enzymatic debridement involves the use of enzymes from outside the body to remove non-living tissue. Debridement enzymes cleave large immobile structural molecules of the cell into smaller molecules that can dissolve and migrate away.

WO 96/24371 A1 discloses an enzyme derived from Antarctic krill, substantially purified, having a purity with respect to macromolecules of at least about 95%, and having multifunctional activity comprising at least one of a chymotrypsin, trypsin, collagenase, elastase or exo peptidase activity, a molecular weight between about 20 kd and about 40 kd as determined by SDS PAGE, and substantial homology to krill-derived multifunctional hydrolase to remove dead tissue from wounds.

The object of the present invention is to provide a further use for a mixture of enzymes from krill and a further method of treatment.

The object is solved by the features of the appended set of claims in which the invention is defined.

According to the invention a mixture of enzymes from Antarctic krill (Euphausia superba) for use in the treatment of a disease related to or an inflammatory condition caused by a biofilm formed on a soft tissue of a mammal, which treatment comprises the treatment of the soft tissue comprising the removal of the biofilm or preventing a formation of the biofilm, wherein the enzymes have molecular weights in the range of 20 kD to 40 kD, as determined by SDS-PAGE, wherein the mixture comprises carbohydrases, lipolytic enzymes, serine proteases with trypsin-like activity or serine proteases with chymotrypsin-like activity, endopeptidases and exopeptidases is provided. The soft tissue is a soft tissue of a mammal on which a biofilm has formed or is expected to form. If a biofilm has formed the treatment comprises the removal of the biofilm and if a biofilm is expected to form the treatment comprises preventing formation of the biofilm. The mixture of enzymes from krill is used in the removal or preventing the formation of the biofilm. Removal of the biofilm usually comprises contacting the biofilm with the mixture of enzymes. Preventing the formation of the biofilm usually comprises contacting a surface with the mixture of enzymes, on which surface the formation of the biofilm is expected to occur. The biofilm forming on soft tissue is different from the salivary proteins comprising biofilm forming plaque on teeth. This is in particular due to the specific surroundings and pH in the oral cavity. A biofilm is a film on a surface comprising specific proteins, other substances, microorganisms acting as a community and extracellular polymeric substances excreted by the microorganisms. A biofilm on a soft tissue usually comprises in addition to these constituents proteins and/or glycoproteins excreted from this soft tissue.

According to Rhoads, D.D. et al. "Evidence of Biofilms in Wounds and the Potential Ramifications", BiofilmClub 2007, pages 1 to 15 biofilms with great species diversity form in wounds of compromised hosts that exhibit an impaired inflammatory response. The poor inflammatory response does not manage the wound's initial bacteria effectively. Consequently, the bacteria can persist within the wound and establish a biofilm community that not only can overcome the host's natural defenses but which is also recalcitrant to antibiotic therapy and other management strategies. The biofilm chronically maintains the wound in an inflammatory phase unless successful therapeutic means can help to eliminate the biofilm infection. The biofilm may even cause or support sepsis, in particular in burn wounds. The biofilm may form before or after a wound has been cleansed of bacteria and debris.

According to James G. A. et al. 2008, Wound Repair and Regeneration 16 (1), pages 37-44 a study comprising 50 chronic wound specimens and 16 acute wound specimens revealed that 60% of the chronic wound specimens and 6% of the acute wound specimens contained a biofilm. Molecular analyses of chronic wound specimens revealed diverse polymicrobial communities and the presence of bacteria, including strictly anaerobic bacteria, not revealed by culture.

The inventors surprisingly found that the biofilms forming on soft tissues can be removed by use of the mixture of enzymes from krill without destroying the soft tissues. They also found that the removal of the biofilm has several advantages in diseases and other conditions they found to be related to biofilms.

The mixture comprises carbohydrases, lipolytic enzymes and/or proteases, serine proteases with trypsin-like activity or chymotrypsin-like activity, endopeptidases and exopeptidases, in particular carboxypeptidases or aminopeptidases. The mixture may be a crude extract from krill comprising proteolytic, lipolytic and carbohydrate splitting activity or an extract from krill more purified than the crude extract such that it has mainly proteolytic activity.

According to an embodiment of the invention the mixture of enzymes has at least two, in particular three, of a chymotrypsin-like, trypsin-like, endo-peptidase or exo-peptidase activity and the mixture of enzymes comprises enzymes having molecular weights in the range of 20 kD to 40 kD, as determined by SDS-PAGE.

The mixture may essentially consist of proteases comprising endopeptidases and exopeptidases.

The biofilm is a biofilm causing an inflammatory condition of said mammal and the treatment comprises a treatment of this condition.

According to one embodiment of the invention the soft tissue is a mucosa, in particular a mucosa damaged by aphtha or Herpes simplex virus infection or mucosa of the urinary tract or mucosa of the gastrointestinal tract, in particular oral mucosa, mucosa of the esophagus or mucosa of the stomach, in particular mucosa of the stomach affected by Helicobacter pylori. The soft tissue may also be an epithelium of skin, in particular of skin affected by a mycosis or a follicular unit. A follicular unit is a naturally-occurring hair follicle or grouping of two to four hair follicles that is extracted together with the hair(s) from a first area of a hair restoration patient to be implanted in a second area of the patient. The follicular unit may be treated with the mixture of enzymes before or even after transplantation. The soft tissue may also be a connective tissue on the surface of a graft, on the surface of a graft contact area of a transplant receipt and/or on the surface of a graft contact area of the graft. The graft may be a skin substitute, in particular a living skin substitute. The inventors found that a biofilm forms on said contact area and that removal of this biofilm prior to transplantation improves access of diverse growth factors and therewith improves the growing together of the graft and the transplant receipt. The graft may also be an organ to be transplanted. In the time between removal of the organ from the donor and inserting it in the recipient a biofilm may form. If a biofilms on such an organ is not removed prior to transplantation the biofilm may expand inside the body after transplantation, in particular due to the immunosuppression of a transplant receiving patient. The soft tissue may also be a connective tissue exposed by surgery. It may also be a chronic or an acute wound or a burn wound.

In another embodiment the soft tissue is the mucosa of the esophagus and the condition is reflux.

According to a further embodiment the soft tissue is oral mucosa and the condition is oral mucositis induced by radiation or chemotherapy.

The treatment may be by oral administration or by topical application, in particular by oral administration or by topical application of a sustained release formulation of the mixture.

The treatment may be by oral administration or by topical application of the mixture at least 4, in particular 6, in particular 7, in particular 8, times per day.

According to one further embodiment the treatment is a treatment for at least 3 days, in particular 4 days, in particular 5 days, in particular one week, in particular two weeks, or a treatment up to one month.

The casein-proteolytic activity of the mixture may be in the range of 0.0003 to 30, in particular in the range of 0.003 to 20, in particular in the range of 0.03 to 10, in particular in the range of 0.3 to 5, units per ml or per g of the mixture.

The krill is Antarctic krill (*Euphausia superba*).

### Examples

### Example 1: Identification of biofilms in chronic wounds by scanning electron microscopy

A patient with a non-healing wound large enough to take several punch biopsies - at least 4 cm x 5 cm - agrees to participate in the experiment. The study is geared towards determining the biofilm structure before and after application of the mixture of enzymes from krill via scanning electron microscopy.

The wound is photographed from a standardized view so that comparison photos over time can be taken. Then 2 wound samples at opposing sides of the wound are taken via punch biopsy and the samples are fixed in commonly accepted procedure. The fixation may be performed by incubation in 2.5% glutaraldehyde in 0.1 M phosphate-buffered solution (PBS), pH 7.2, followed by washing 3 times in PBS, and dehydration. The samples are stuck with double-sided tape to specimen stubs, followed by gold-platinum (50:50) ion coating. To improve the sample mounting the dorsal sides of the samples can be removed. Samples are visualized using a standard scanning electron microscope (SEM) that can be operated at a scanning voltage of 6 kV - 10 kV. See also: James G. A. et al. 2008, Wound Repair and Regeneration 16 (1), pages 37-44.

Immediately following this initial "baseline" sampling the patient is scheduled to receive 5 applications of a solution of the mixture of enzymes from krill according to the invention in 2 hour intervals for 3 days, a total of 15 applications. The solution of the mixture of enzymes from krill to be used may be prepared as disclosed in EP 0 107 634 A2 or the standard Krillase^{®} stock product purchased from Arcimboldo AB, Kräftriket 11, SE-104 05 Stockholm, Sweden as a vial containing 60 casein units of freeze-dried Euphausia extract powder and reconstituted in 10 ml sterile 0.9% sodium chloride solution. For the application sterile gauze is shaped according to the wound, soaked with the solution, and applied to the wound. The wound area is thereafter covered with occlusive film to prevent drying. After every 3rd application pictures are taken.

Two hours after the last application of the enzyme mixture solution 2 more punch biopsies are taken in proximity of first 2 biopsy areas. The samples are treated as described above.

Results: The first set of SEM pictures will clearly show a polymicrobial biofilm. The second set of pictures will show that the appearance of the wound is improving with increased enzyme mixture application number. The last set of SEM photographs will clearly show a clean wound and lack of organized biofilm. The patient will be admitted for a grafting procedure.

### Example 2: Histological Analysis

Two patients agree to participate in a study with the objective to determine biofilm existence via standard histological analysis before and after application of the mixture of enzymes according to the invention. Each of them has a non-healing wound large enough to take several punch biopsies (at least 4 cm x 5 cm). The wounds of the 2 patients are photographed from a standardized view so that comparison photos over time can be taken. Then each wound of the two patients is sampled at opposing sides of the wound via punch biopsy. The excised samples are prepared for histological analysis by bisecting at their largest diameter for hematoxylin and eosin staining (HE). The tissues are then fixed in formalin, embedded in paraffin, and cut into 4-pm sections. Paraffin is removed with a xylene wash, followed by a standard hematoxylin and eosin staining protocol to prepare samples for analysis under a light microscope. The slides are examined using a digital analysis system by a masked observer evaluating all histological sections.

Immediately following this initial "baseline" sampling the patients are scheduled to receive 5 applications of a solution of the mixture of enzymes from krill according to the invention in 2 hour intervals for 3 days, a total of 15 applications. The solution of the mixture of enzymes from krill to be used may be the same as described for example 1. Application of the solution is as described for example 1. After every 3rd application pictures are taken.

Two hours after the last application of the enzyme mixture solution 2 more punch biopsies are taken in proximity of first 2 biopsy areas. The samples are treated as described above.

Results: The first set of slides for the first patient will clearly show a polymicrobial biofilm, while there will be no discernible bacterial colony for the second patient. The second set of pictures will show for both patients the appearance of the wound is improving with increasing enzyme mixture application number. In the case of the first patient a degradation of the biofilm and in addition the known debridement of the wound occurs whereas in the case of the second patient only the known debridement occurs. The last set of slides will show lack of organized biofilm for both patients and clean wounds.

### Example 3: Microbial Sequencing

Biofilms are polymicrobial and for antibiotic treatment therapy it is of value to know what the pathogens are and in what relative percentage they are in the infected area.

About 10 patients agree to participate to study wound bacteria compositions via DecodEX^{™} Microbial Sequencing offered by PathoGenius^{®} Laboratories, 1004 Garfield Dr., Building #340, Lubbock, Texas 79416, before and after application of the mixture of enzymes from krill according to the invention. Each of them has a non-healing wound large enough to take several punch biopsies (at least 4 cm x 5 cm). The wounds of the 10 patients are photographed from a standardized view so that comparison photos over time can be taken. Then each wound of the patients is sampled following the instruction provided by Pathogenius Laboratories.

In general: 1. Obtain a sample using a curette or other debridement tools. 2. Place the sample (tissue or entire applicator tip) into the provided containers, 3. Send samples in provided shipment kit containers to Pathogenius Laboratories.

Immediately following this initial "baseline" sampling the patients are scheduled to receive 5 applications of a solution of the mixture of enzymes from krill according to the invention in 2 hour intervals for 3 days, a total of 15 applications. The solution of the mixture of enzymes from krill to be used may be the same as described for example 1. Application of the solution is as described for example 1. After every 3rd application pictures are taken.

Two hours after the last application of the enzyme mixture solution each patient's wound is sampled again in proximity of the first 2 sampling sites. The samples are treated as described above.

Results: A first set of tables will show that the majority of patients has a polymicrobial biofilm infection, however some patients do not show polymicrobial compositions. A following set of pictures will show for all patients that the appearance of the wound is improving with increasing enzyme mixture application number. A last set of tables will show that the degree of polymicrobial contamination of the wounds is drastically reduced or the polymicrobial contamination is eliminated.

### Example 4: Biofilms identification via Swabbing and Microbial Sequencing

A patient with a small wound (anal fissure) that cannot be sampled by taking several biopsies out of the wound agrees to participate in the experiment. The study is geared towards determining the biofilm composition in a small wet wound before and after application of the mixture of enzymes from krill according to the invention via swabbing and microbial sequencing of the collected wound surface fluid.

"24-Hour Rapid Screening" sample kits from Pathogenius Laboratories will be used to examine the specimen for the most commonly identified microbes, including Staphylococcus (including MRSA), Streptococcus, Haemophilus, Enterococcus (including VRE), Pseudomonas, Serratia, Candida, and many more.

The patient is instructed to defecate and clean the area as unusual. Then 2 hours after the cleaning, the anal fissure of the patient will be photographed from a standardized view so that comparison photos over time can be taken. Then the anal mucosa wound surface of the patient is sampled following the instructions provided by Pathogenius Laboratories (common swabbing techniques). In general: 1. Obtain a sample using a sterile cotton-tipped applicator. 2. Place the sample entire applicator tip into the provided containers, 3. Send samples in provided shipment kit containers to Pathogenius Laboratories.

Immediately following this initial "baseline" sampling the patient is scheduled to receive 6 applications of a solution of the mixture of enzymes from krill according to the invention in 1 hour intervals. During that time the patient is not allowed to defecate. The solution of the mixture of enzymes from krill to be used may be the same as described for example 1. For the application sterile gauze is soaked with the solution and applied to the wound. After every 3rd application pictures are taken.

One hour after the last application of the enzyme mixture solution anal fissure wound is sampled again as described above.

Results: The first table will show the baseline bacterial composition of the wound, which is indicative of a polymicrobial biofilm infection. A following set of pictures will show that the appearance of the wound is improving with increasing enzyme mixture application number. A last table will show that the degree of the polymicrobial infection is drastically reduced as well as the number of bacterial species in the wound is lower than before.

### Example 5: Use of the mixture of enzymes from krill according to the invention for preventing the formation of a biofilm on an organ to be transplanted

Immediately after removal of an organ from a donor which organ is intended to be transplanted a biofilm may form on its surface. To prevent formation of the biofilm the organ is put in a sterile 0.9% sodium chloride solution comprising the mixture of enzymes from krill according to the invention immediately after removal from the donor. To keep the organ vital it is kept in this solution at about 4°C during its transport to the recipient. The enzymes from krill are predestinated for such an application because they are still active at 4°C. A further advantage is that it is not required to remove residual enzyme solution from the surface of the organ before transplantation because the enzymes are very quickly inactivated in the human body.

### Example 6: Use of the mixture of enzymes from krill according to the invention for removal of a Helicobacter pylori (H. pylori) biofilm

Resistance of H. pylori infection to conventional therapies is frequently associated with biofilm formation. Biofilms formed by H. pylory are complex microbiological ecosystems surrounding the bacteria and allowing proliferation in the hostile environment of the stomach. H. pylori biofilms on human gastric mucosa has been proven to be the causative agent of gastric ulcers.

To degrade the H. pylori biofilm patients are treated for 2 days with omeprazole to normalize their gastric pH. Thereafter treatment with omeprazole is maintained and in addition patients are treated with the mixture of enzymes from krill according to the invention by administration of gelatin capsule comprising the mixture of enzymes 3 times a day for 7 days. Biofilm removal is followed in parallel by sampling of gastric biopsies and examination of the biopsies for the presence of the biofilm. Examination may be performed by scanning electron microscopy as described in example 1. Example 7: Use of the mixture of enzymes from krill according to the invention for the treatment of follicular units after transplantation

A patient's skin area with freshly transplanted follicular units is treated by applying the mixture of enzymes from krill according to the invention. The area comprises a small wound at every site where a follicular unit has been inserted. A solution of the mixture of enzymes from krill to be used may be the same as described for example 1. For the application sterile gauze is soaked with the solution and applied to the area 3 times per day for 7 days. Due to this application biofilms formed at the follicular units and/or the wounds are removed and/or the formation of biofilm at the follicular units and/or the wounds is prevented resulting in a relatively fast healing without significant scar formation and without significant loss of freshly implanted hair.

## Claims

1. Mixture of enzymes from Antarctic krill (Euphausia superba) for use in the treatment of a disease related to or an inflammatory condition caused by a biofilm formed on a soft tissue of a mammal, which treatment comprises the treatment of the soft tissue comprising the removal of the biofilm or preventing a formation of the biofilm, wherein the enzymes have molecular weights in the range of 20 kD to 40 kD, as determined by SDS-PAGE, wherein the mixture comprises carbohydrases, lipolytic enzymes, serine proteases with trypsin-like activity or serine proteases with chymotrypsin-like activity, endopeptidases and exopeptidases.

2. Mixture for use according to claim 1 , wherein the soft tissue is epithelial tissue or mucosa.

3. Mixture for use according to any of the preceding claims, wherein the soft tissue is a mucosa damaged by aphtha or Herpes simplex virus infection or mucosa of the urinary tract, mucosa of the gastrointestinal tract, oral mucosa, mucosa of the esophagus, mucosa of the stomach, mucosa of the stomach affected by Helicobacter pylori, an epithelium of skin, an epithelium of skin affected by a mycosis or a follicular unit, or a connective tissue on the surface of a graft, on the surface of a graft contact area of a transplant receipt and/or on the surface of a graft contact area of the graft or a connective tissue exposed by surgery or a chronic or an acute wound, an anal fissure, or a burn wound.

4. Mixture for use according to claim 3, wherein the graft is a skin substitute or a living skin substitute.

5. Mixture for use according to claim 3, wherein the soft tissue is the mucosa of the esophagus and wherein the disease or condition is reflux.

6. Mixture for use according to claim 3, wherein the soft tissue is oral mucosa and the disease or condition is oral mucositis induced by radiation or chemotherapy.

7. Mixture for use according to claim 3, wherein the soft tissue is oral mucosa and the disease or condition is aphtha.

8. Mixture for use according to any of the preceding claims, wherein the treatment is by oral administration or by topical application.

9. Mixture for use according to claim 8, the treatment is by oral administration or topical application of a sustained release formulation of the mixture.

10. Mixture for use according to claim 8 or 9, wherein the treatment is by oral administration or by topical application of the mixture at least 4, 6, 7, or 8 times per day.

11. Mixture for use according to any of the preceding claims, wherein the treatment is a treatment for at least 3 days, 4 days, 5 days, one week, or two weeks, or a treatment up to one month.

12. Mixture for use according to any of the preceding claims, wherein the casein-proteolytic activity of the mixture is in the range of 0.0003 to 30, in the range of 0.003 to 20, in the range of 0.03 to 10, or in the range of 0.3 to 5 units per ml or per g of the mixture.

## Patentansprüche

1. Enzymmischung von antarktischem Krill (Euphausia superba) zur Verwendung bei der Behandlung eines entzündlichen Zustands, der durch einen auf einem Weichgewebe eines Säugers gebildeten Biofilm verursacht wird, oder einer Erkrankung, die damit im Zusammenhang steht, wobei die Behandlung die Behandlung des Weichgewebes umfasst, umfassend die Entfernung des Biofilms oder ein Verhindern einer Bildung des Biofilms, wobei die Enzyme Molekulargewichte in dem Bereich von 20 kD bis 40 kD aufweisen, wie durch SDS-PAGE bestimmt, wobei die Mischung Carbohydrasen, lipolytische Enzyme, Serinproteasen mit trypsinähnlicher Aktivität oder Serinproteasen mit chymotrypsinähnlicher Aktivität, Endopeptidasen und Exopeptidasen umfasst.

2. Mischung zur Verwendung nach Anspruch 1, wobei das Weichgewebe Epithelgewebe oder Schleimhaut ist.

3. Mischung zur Verwendung nach einem der vorstehenden Ansprüche, wobei das Weichgewebe eine Schleimhaut, die durch Aphthen oder Herpessimplex-Virusinfektion geschädigt ist, oder Schleimhaut des Harntrakts, Schleimhaut des Magen-Darm-Trakts, Mundschleimhaut, Schleimhaut der Speiseröhre, Magenschleimhaut, Magenschleimhaut, die von Helicobacter pylori betroffen ist, ein Hautepithel, ein Hautepithel, das von einer Mykose oder einer follikulären Einheit betroffen ist, oder ein Bindegewebe auf der Oberfläche eines Transplantats, auf der Oberfläche eines Transplantat-Kontaktbereichs eines Transplantationserhalts und/oder auf der Oberfläche eines Transplantat-Kontaktbereichs des Transplantats oder ein Bindegewebe, das durch Operation oder eine chronische oder eine akute Wunde, eine Analfissur oder eine Brandwunde freigelegt ist, ist.

4. Mischung zur Verwendung nach Anspruch 3, wobei das Transplantat ein Hautersatz oder ein lebender Hautersatz ist.

5. Mischung zur Verwendung nach Anspruch 3, wobei das Weichgewebe die Schleimhaut der Speiseröhre ist, und wobei die Erkrankung oder der Zustand Reflux ist.

6. Mischung zur Verwendung nach Anspruch 3, wobei das Weichgewebe Mundschleimhaut ist und die Erkrankung oder der Zustand orale Mukositis ist, die durch Bestrahlung oder Chemotherapie induziert ist.

7. Mischung zur Verwendung nach Anspruch 3, wobei das Weichgewebe Mundschleimhaut ist und die Erkrankung oder der Zustand Aphthen ist.

8. Mischung zur Verwendung nach einem der vorstehenden Ansprüche, wobei die Behandlung durch orale Verabreichung oder durch topische Anwendung erfolgt.

9. Mischung zur Verwendung nach Anspruch 8, wobei die Behandlung durch orale Verabreichung oder topische Anwendung einer Formulierung mit verzögerter Freisetzung der Mischung erfolgt.

10. Mischung zur Verwendung nach Anspruch 8 oder 9, wobei die Behandlung durch orale Verabreichung oder durch topische Anwendung der Mischung mindestens 4, 6, 7 oder 8 Mal täglich erfolgt.

11. Mischung zur Verwendung nach einem der vorstehenden Ansprüche, wobei die Behandlung eine Behandlung für mindestens 3 Tage, 4 Tage, 5 Tage, eine Woche oder zwei Wochen oder eine Behandlung bis zu einem Monat ist.

12. Mischung zur Verwendung nach einem der vorstehenden Ansprüche, wobei die Kasein-proteolytische Aktivität der Mischung in dem Bereich von 0,0003 bis 30, in dem Bereich von 0,003 bis 20, in dem Bereich von 0,03 bis 10 oder in dem Bereich von 0,3 bis 5 Einheiten pro ml oder pro g der Mischung liegt.

## Revendications

1. Mélange d'enzymes à partir de krill antarctique (Euphausia superba) pour une utilisation dans le traitement d'une maladie liée à ou d'une affection inflammatoire provoquée par un biofilm formé sur un tissu mou d'un mammifère, lequel traitement comprend le traitement du tissu mou comprenant le retrait du biofilm ou la prévention d'une formation du biofilm, les enzymes ayant des poids moléculaires dans la plage de 20 kD à 40 kD, tel que déterminé par SDS-PAGE, le mélange comprenant des carbohydrases, des enzymes lipolytiques, des sérine protéases avec une activité de type trypsine ou des protéases de sérine avec l'activité de type chymotrypsine, des endopeptidases et des exopeptidases.

2. Mélange pour une utilisation selon la revendication 1, dans lequel le tissu mou est un tissu épithélial ou une muqueuse.

3. Mélange pour une utilisation selon l'une quelconque des revendications précédentes, dans lequel le tissu mou est une muqueuse endommagée par une infection par le virus de l'aphte ou de l'herpès simplex ou une muqueuse des voies urinaires, une muqueuse du tractus gastro-intestinal, une muqueuse buccale, une muqueuse de l'œsophage, une muqueuse de l'estomac, une muqueuse de l'estomac affecté par Helicobacter pylori, un épithélium de peau, un épithélium de peau affecté par une mycose ou une unité folliculaire, ou un tissu conjonctif sur la surface d'un greffon, sur la surface d'une zone de contact de greffe d'un reçu de greffe et/ou sur la surface d'une zone de contact de greffe du greffon ou d'un tissu conjonctif exposé par chirurgie ou plaie chronique ou aiguë, une fissure anale ou une plaie de brûlure.

4. Mélange pour une utilisation selon la revendication 3, dans lequel le greffon est un substitut de la peau ou un substitut de la peau vivante.

5. Mélange pour une utilisation selon la revendication 3, dans lequel le tissu mou est la muqueuse de l'œsophage et la maladie ou la condition étant un reflux.

6. Mélange pour une utilisation selon la revendication 3, dans lequel le tissu mou est une muqueuse buccale et la maladie ou lac condition est une muqueuse buccale induite par un rayonnement ou une chimiothérapie.

7. Mélange pour une utilisation selon la revendication 3, dans lequel le tissu mou est la muqueuse buccale et la maladie ou la condition est l'aphte.

8. Mélange pour une utilisation selon l'une quelconque des revendications précédentes, dans lequel le traitement est par administration orale ou par application topique.

9. Mélange pour une utilisation selon la revendication 8, le traitement étant par administration orale ou application topique d'une formulation à libération prolongée du mélange.

10. Mélange pour une utilisation selon la revendication 8 ou 9, dans lequel le traitement est par administration orale ou par application topique du mélange au moins 4, 6, 7 ou 8 fois par jour.

11. Mélange pour une utilisation selon l'une quelconque des revendications précédentes, dans lequel le traitement est un traitement pendant au moins 3 jours, 4 jours, 5 jours, une semaine ou deux semaines, ou un traitement jusqu'à un mois.

12. Mélange pour une utilisation selon l'une quelconque des revendications précédentes, dans lequel l'activité caséine-protéolytique du mélange est dans la plage de 0,0003 à 30, dans la plage de 0,003 à 20, dans la plage de 0,03 à 10, ou dans la plage de 0,3 à 5 unités par ml ou par g du mélange.
